# DEMANDE DE BREVET EUROPEEN

(11) **EP 4 628 090 A1**
(43) Date de publication de la demande: **08.10.2025**
(21) Numéro de dépôt: 24168023.0
(22) Date de dépôt: 02.04.2024
(51) Int. Cl.: A61K 36/14, A61K 36/53, A61K 36/87, A61P 17/02, A61P 25/02, A61P 39/00, A61K 9/00, A61K 9/06, A61K 9/10

(54) **PRINCIPE ACTIF COPRENANT UN EXTRAIT CONSTITUE DE JUNIPERUS COMMUNIS, ROSMARINUS OFFICINALIS ET VIN BLANC, ET SON UTILISATION MEDICALE.**

(71) Demandeur: Umbria Care, 13100 Aix-en-Provence (FR)
(72) Inventeur: MORI, Serge, 13100 LE THOLONET (FR)
(74) Mandataire: Aquinov

(57) **Abrégé**

La présente invention se rapporte au domaine de la cosmétique et/ou pharmaceutique en proposant un principe actif comprenant un extrait composé d'un mélange d'ingrédients naturels ayant des vertus particulièrement d'intérêts pour lutter et soulager les douleurs, en particulier les douleurs liées à la sciatique. Ainsi, l'invention porte principalement sur un principe actif comprenant un extrait constitué d'un mélange de baies de genièvre, de romarin et de vin blanc.

## Description

### Domaine technique

La présente invention se rapporte au domaine de la cosmétique et/ou pharmaceutique en proposant un principe actif comprenant un extrait issu d'un mélange d'ingrédients naturels ayant des vertus particulièrement d'intérêts pour lutter et soulager les douleurs, en particulier les douleurs liées à la sciatique. L'invention porte ainsi principalement sur un principe actif comprenant un extrait constitué essentiellement d'un mélange de baies de genièvre, de romarin et de vin blanc.

### Etat de l'art

Les consommateurs sont de plus en plus soucieux des produits qu'ils consomment. Ils sont donc à la recherche de produits formulés à partir d'ingrédients d'origine naturelle, respectueux de l'environnement et de l'utilisateur, et ce, afin de refléter leurs préoccupations environnementales et éthiques, ainsi que leurs aspirations à un mode de vie plus sain.

De tels produits s'affranchissent donc d'ingrédients issus de la pétrochimie ou encore de molécule de synthèse, ce qui permet avantageusement de limiter les effets secondaires indésirables tels que les sensations d'inconfort, l'apparition de rougeurs. En outre, ces produits sont d'avantage éco-responsables.

Dans ce contexte, les produits à base d'ingrédients naturels d'origine végétale, respectueux de l'environnement, constituent une forte attente de la part des consommateurs. La présente invention s'inscrit particulièrement dans cette démarche et vise à proposer un produit naturel pour lutter, soulager les douleurs, notamment les douleurs liées à la sciatique.

La sciatique est causée par l'irritation du nerf sciatique, celui-ci descend le long de la jambe jusqu'au pied. La sciatique est la manifestation d'une douleur intense au niveau d'un membre inférieur, souvent associé à des douleurs lombaires. La sciatique peut avoir plusieurs causes et est présente aussi bien chez les personnes jeunes, que les personnes âgées.

Elle peut être due à une hernie discale, un traumatisme de la région lombaire, une maladie inflammatoire, ou encore une réduction du canal lombaire, par exemple causée par l'arthrose.

Bien que douloureuse, peu de solutions existent pour soulager de manière efficace les douleurs et limiter les risques de récidives. Lorsque que ces douleurs sont supportables, il est conseillé de les soulager par des exercices ou mouvements spécifiques, l'utilisation de la chaleur permet également de détendre les muscles contractées. Les seules solutions pharmaceutiques connues, à ce jour, reposent sur la prise d'antalgiques, tels que le paracétamol ou les anti-inflammatoires non stéroïdiens.

Dans ce contexte, il existe un besoin pour de nouveaux produits cosmétiques et/ou thérapeutiques, à application topique, à base d'ingrédients naturels pour soulager les douleurs, en particulier les douleurs de la sciatique.

### Résumé de l'invention

Pour répondre à ce besoin, les inventeurs se sont intéressés à un mélange de plantes et d'extraits de plantes, à savoir un mélange de baies de genièvres et de romarin, ayant macéré dans du vin blanc pour préparer un produit de soin naturel. De façon surprenante, les inventeurs ont constaté que ce mélange de plantes et extraits de plantes spécifiques permet de diminuer les douleurs, et ce seulement quelques jours après l'application du produit.

Pris individuellement, chacun des ingrédients est connu pour avoir des vertus cosmétiques et/ou thérapeutiques diverses. On peut notamment citer des effets antioxydants, antirhumatismaux, anti-inflammatoires etc. Toutefois, la combinaison spécifique de ces ingrédients naturels n'est pas connue.

Ainsi, l'invention concerne un principe actif comprenant un extrait hydroalcoolique, ledit extrait comprenant un mélange constitué essentiellement de :
- Fruits de *Juniperus Communis,*
- *Rosmarinus Officinalis,* et
- vin blanc à 12°C

Préférentiellement, le principe actif selon l'invention comprend un extrait hydroalcoolique constitué essentiellement de :
- Fruits de *Juniperus Communis,*
- *Rosmarinus Officinalis,* et
- vin blanc à 12°C.

Très préférentiellement, le mélange constitué essentiellement des fruits de *Juniperus Communis, Rosmarinus Officinalis,* et de vin blanc à 12°C, constitue l'extrait et le principe actif selon la présente invention. Bien entendu, des excipients peuvent éventuellement être ajoutés au principe actif, afin d'en améliorer sa galénique et faciliter son administration ou son application.

Selon un autre objet préféré de la présente invention, les fruits de *Juniperus Communis* représentent entre 0,5 et 2% en poids du poids total du mélange. Selon un autre objet préféré, *Rosmarinus Officinalis* représente entre 1,5 et 8% en poids du poids total du mélange. Enfin, le vin blanc représente préférentiellement entre 90 et 98% en poids du poids total du mélange.

Dans le contexte de la présente invention, les branches et feuilles de *Rosmarinus Officinalis* sont préférées pour améliorer encore l'efficacité du principe actif selon l'invention. Aussi, le principe actif selon l'invention est avantageusement d'origine naturelle.

Enfin, l'invention se rapporte également à un procédé de d'extraction, ledit procédé comprenant au moins les étapes suivantes :
a. Mélanger les fruits de *Juniperus Communis, Rosmarinus Officinalis,* et le vin blanc
b. Macération dans le vin blanc à une température comprise entre 25° et 60°C,
c. Filtration du macérat obtenu, formant l'extrait,
d. Eventuellement concentration et/ou purification de l'extrait obtenu à l'étape précédente.

L'extrait obtenu formant ainsi le principe actif selon la présente invention. Ainsi, selon un autre objet, l'invention porte sur un principe actif obtenu par ce procédé d'extraction.

Selon un autre aspect de l'invention, les inventeurs ont constaté que le principe actif présentait de nombreuses vertus, à savoir, il présente avantageusement un effet anti-inflammatoire et permet de lutter et/ou soulager les douleurs, en particulier les douleurs liées à la sciatique, permettant ainsi de répondre aux problématiques de l'art antérieur. En outre, il présente des effets apaisants, relipidants, et améliore également la fonction barrière. Enfin, il présente également des effets bénéfiques sur la cicatrisation.

Aussi, il peut être utilisé pour des applications cosmétiques ou thérapeutiques et être ainsi intégré dans une composition cosmétique ou thérapeutique, avantageusement une composition sous une forme adaptée à une application topique.

Préférentiellement, la composition selon la présente invention comprend au moins un excipient acceptable, à savoir un excipient cosmétiquement ou pharmaceutiquement acceptable, par exemple un gélifiant.

Ainsi, selon un autre objet préféré, la présente composition comprend entre 0,5 et 3% d'au moins un gélifiant, en poids du poids total de la composition. Plus préférentiellement, la présente composition comprend entre 0,9 et 1,5% d'au moins un gélifiant. Ledit gélifiant est préférentiellement un carbomère permettant d'obtenir une viscosité satisfaisante de l'extrait selon l'invention. Préférentiellement, viscosité de la composition selon l'invention est comprise entre 20 000 et 30 000 cps. Une telle viscosité est satisfaisante pour formuler préférentiellement le produit sous forme de gel ou de crème destiné à être appliqué topiquement.

A cet effet, la présente composition se présente avantageusement sous une forme choisie parmi un gel, une crème, un sérum, un masque et une lotion, plus préférentiellement un gel ou une crème.

Lorsque les personnes souffrent de douleurs, notamment de douleurs liées à la sciatique, le principe actif selon l'invention ou la composition le comprenant vise à être utilisé comme médicament. Préférentiellement, pour lutter contre l'inflammation et/ou contre les douleurs de la sciatique. Selon une variante surprenante, la présente invention vise également à améliorer la cicatrisation cutanée.

Enfin, selon un autre aspect, l'invention se rapporte également à une utilisation cosmétique du principe actif selon l'invention ou d'une composition le comprenant, en particulier pour ses effets apaisants, relipidants, mais également pour améliorer, renforcer la barrière cutanée chez des personnes ne souffrant pas de sciatique.

D'autres caractéristiques et avantages ressortiront de la description détaillée de l'invention, des exemples et des figures qui vont suivre.

### Brève description des Figures

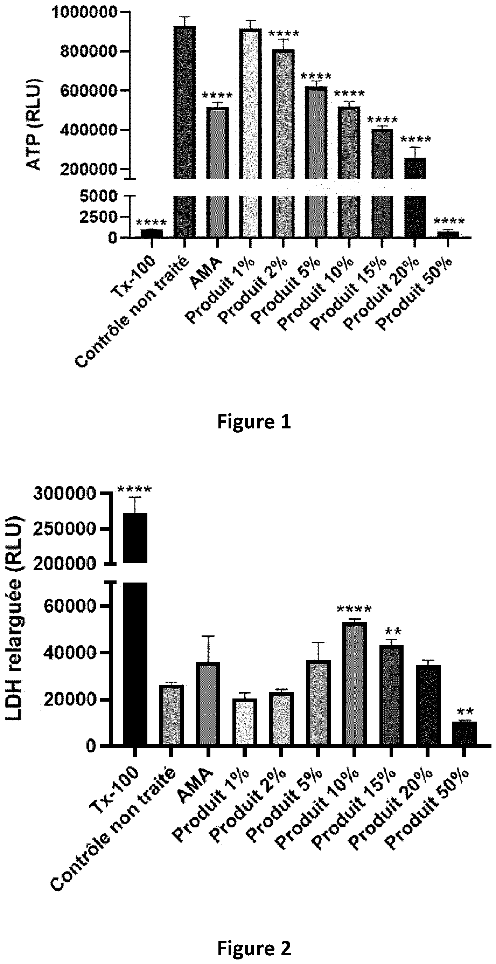

[Fig. 1] représente les résultats de la teneur en ATP des kératinocytes après traitement par l'extrait selon l'invention à des doses croissantes (1%-50%) en comparaison avec l'inhibiteur Antimycine A (AMA). Les données sont présentées comme la moyenne en unités relatives de lumière (RLU) ± SD, *p < 0,05 par rapport au groupe de contrôle NT.
[Fig. 2] représente les résultats de la mesure de LDH des kératinocytes après traitement par l'extrait à doses croissantes (1%-50%) en comparaison avec un traitement au Triton X-100 (Tx-100, LDH maximale). Les données sont présentées comme la moyenne en unités relatives de lumière (RLU) ± SD, *p < 0,05 par rapport au groupe de contrôle NT.

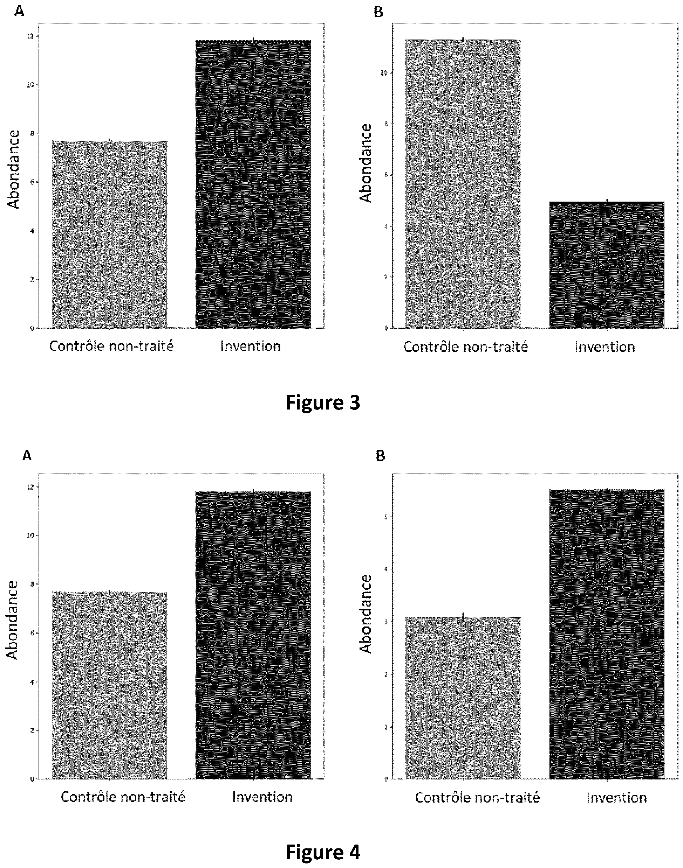

[Fig. 3] représente sous forme d'histogramme, la synthèse des changements présentés dans le réseau des protéines impliquées dans la réparation (cicatrisation) de la peau. Les données sont présentées comme le % de changement de l'abondance de protéines associées au cluster « cicatrisation » par rapport au groupe de contrôle NT.
[Fig. 4] représente sous forme d'histogramme, la synthèse des changements présentés dans le réseau apaisant de la peau. Les données sont présentées comme le % de changement de l'abondance de protéines associées au cluster « apaisant » par rapport au groupe de contrôle NT.

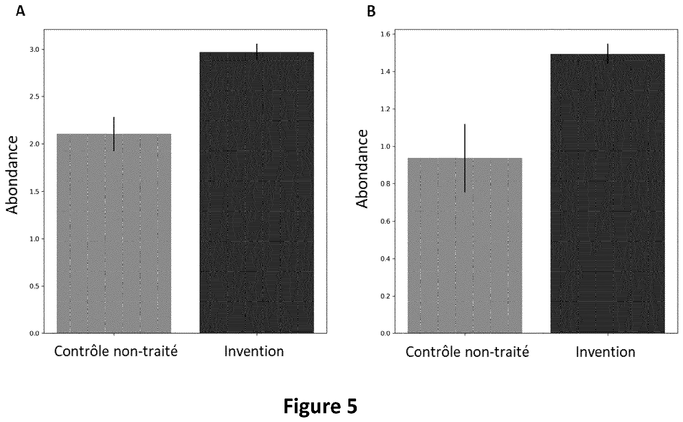

[Fig. 5] représente sous forme d'histogramme la synthèse des changements présentés dans le réseau relipidant de la peau. Les données sont présentées comme le % de changement de l'abondance de protéines associées au cluster « relipidant » par rapport au groupe de contrôle NT.

### Description détaillée de l'invention

### Définition

Par « principe actif » au sens de l'invention, on entend un extrait obtenu à partir d'un mélange d'ingrédients naturels, notamment *Juniperus Communis, Rosmarinus officinalis* et du vin blanc, ledit extrait comprenant au moins un ensemble de molécules présentant un effet cosmétique et/ou thérapeutique en application topique. Préférentiellement, l'effet cosmétique sur la peau est un effet apaisant et l'effet thérapeutique soulage les douleurs, notamment les douleurs liées à la sciatique.

Par « extrait de *Juniperus Communis* et *Rosmarinus officinalis* et de vin blanc » au sens de l'invention, on entend un extrait comprenant au moins un ensemble de molécules issu préférentiellement des baies de *Juniperus Communis* et des branches et feuilles de *Rosmarinus officinalis,* ayant macéré dans du vin blanc, ledit vin blanc présentant un degré alcoolique compris entre 11° et 13°, avantageusement 12°. L'extrait selon l'invention n'est pas une huile essentielle.

### Principe actif

La présente invention a donc pour objet un principe actif comprenant un extrait hydroalcoolique, ledit extrait comprenant un mélange de :
- Fruits de *Juniperus Communis,*
- *Rosmarinus Officinalis,* et
- vin blanc à 12°C

Les fruits de *Juniperus Communis,* ou baies de genièvre sont issus d'un petit arbre épineux, de la famille des cyprès, le genévrier (*Juniperus communis*)*.* Originaire d'Inde, ses baies qui sont en réalité des pseudofruits, qui mettent trois ans à mûrir, passant du vert clair au noir. Ces baies sont connues pour servir à parfumer divers alcools, tel que le vin de genièvre, le gin, l'aquavit, ou encore le jenever.

L'huile essentielle de genévrier, en application locale, est connue pour soulager les douleurs. Enfin, les huiles issues des baies sont également utilisées pour traiter des désordres fréquents tels que l'acné, le psoriasis, les pellicules et l'arthrite des articulations, car elles permettent de réguler la quantité de graisse de la peau.

Le romarin (*Rosmarinus officinalis*) est un arbrisseau des régions méditerranéennes poussant sur des sols calcaires, à l'état sauvage, ou cultivé. Il fait partie de la famille des lamiacées et ses feuilles renferment une huile essentielle. En phytothérapie, on utilise les sommités fleuries entières séchées ou l'huile essentielle. On lui prête de nombreuses vertus, par exemple pour aider à soulager améliorer la mémoire, stimuler le système immunitaire et circulatoire et améliorer la croissance des cheveux. Il peut être infusé avant utilisé en externe. Enfin, l'huile essentielle de romarin est connue pour soulager les entorses.

Enfin, le vin et plus largement des extraits issus de la vigne *Vitis vinifera* contiennent des principes actifs aux propriétés anti-âge, éclat, anti-oxydante. Dans le contexte de l'invention, le vin est avantageusement un vin blanc sec présentant un degré alcoolique de l'ordre de 12°.

L'ensemble de ces ingrédients naturels sont en outre, d'origine biologique, là encore pour réduire les risques d'inconfort, d'irritation, susceptible d'être occasionné par des ingrédients comprenant des traces de métaux, pesticides, etc.

Ainsi, l'extrait selon la présente invention présente avantageusement une naturalité supérieure à 96% selon la norme ISO 16 128 dont 94% d'ingrédients d'origine biologique.

Ces ingrédients pris individuellement sont connus pour avoir de nombreuses vertus thérapeutiques ou cosmétiques. A la différence de certaines utilisations décrites dans l'art antérieur, l'extrait selon l'invention n'est et ne comprend pas d'huile essentielle. Ainsi, l'extrait selon l'invention est préférentiellement un extrait hydroalcoolique.

Préférentiellement, le principe actif selon la présente invention comprend un extrait hydroalcoolique constitué essentiellement de :
- Fruits de *Juniperus Communis,*
- *Rosmarinus Officinalis,* et de
- vin blanc à 12°C

De façon surprenante, les inventeurs ont constaté que l'extrait selon l'invention permettait de soulager les douleurs et notamment les douleurs liées à la sciatique.

Ainsi, l'extrait selon l'invention soulage les douleurs musculaires et articulaires seulement quelques jours après application topique du principe actif comprenant l'extrait selon l'invention.

D'autre part, les inventeurs ont également démontré à travers des analyses protéomiques que l'association de ces ingrédients constituant l'extrait selon l'invention présente des effets pour apaiser la peau, améliorer la cicatrisation, tout en aidant à maintenir une fonction barrière optimale. Ceci améliorant également l'hydratation cutanée.

L'efficacité est encore améliorée lorsque l'extrait constituant le principe actif selon l'invention comprend un mélange dans lequel, les fruits de *Juniperus Communis* représentent entre 0,5 et 2% en poids du poids total du mélange, et/ou *Rosmarinus Officinalis* représente entre 1,5 et 8% en poids du poids total du mélange, et/ou le vin blanc représente entre 90 et 98% en poids du poids total du mélange.

Avantageusement le mélange constituant l'extrait selon la présente invention est issu des feuilles et branches de *Rosmarinus Officinalis.*

Selon un autre objet, l'invention se rapporte également à un procédé de préparation du principe actif selon l'invention, comprenant préférentiellement les étapes suivantes :
a. Mélange des fruits de *Juniperus Communis, Rosmarinus Officinalis,* dans du vin blanc
b. Macération des fruits de *Juniperus Communis* et de *Rosmarinus Officinalis* dans le vin blanc à une température comprise entre 25° et 60°C,
c. Filtration du macérat obtenu, formant l'extrait
d. Eventuellement concentration et/ou purification de l'extrait obtenu à l'étape précédente.

Le principe actif cosmétique selon l'invention se présente alors, avantageusement, sous forme liquide. Lorsqu'il se présente sous forme liquide, le principe actif selon l'invention est préférentiellement l'extrait tel que décrit précédemment en tant que tel. Il peut être coloré et/ou être décoloré par tout procédé connu de l'homme du métier. Le principe actif peut éventuellement être combiné à des agents de stabilisation ou à des agents de conservation.

Le principe actif selon l'invention peut avantageusement être intégré dans une composition, celle-ci comprenant alors le principe actif selon l'invention et au moins un excipient acceptable.

Ainsi, lorsqu'une forme gélifiée est souhaitée, le principe actif selon l'invention peut être formulé avec au moins un gélifiant afin d'obtenir une composition sous forme une forme gélifiée. Une telle forme est particulièrement d'intérêt pour pouvoir être appliquée localement sur la peau afin de soulager les douleurs localement, notamment les douleurs liées à la sciatique.

La présente composition se présente préférentiellement sous une forme adaptée à une application topique. Plus préférentiellement, ladite composition se présente sous une forme choisie parmi un gel, une crème, un sérum, un masque et une lotion. Très préférentiellement, sous une forme de gel ou de crème.

Lorsque la composition se présente sous forme de gel ou de crème, la composition comprend le principe actif selon l'invention constitué par le mélange de fruits de *Juniperus Communis, Rosmarinus Officinalis,* et de vin blanc à 12°C, et en outre au moins un agent gélifiant. Préférentiellement l'agent gélifiant est un polymère d'acrylate, plus préférentiellement un carbomère.

Les carbomères sont des polymères d'acide acrylique (acide 2-propénoïque), de très hauts poids moléculaires, utilisés dans le domaine médical ou cosmétique, en raison de leur propriétés bioadhésives et épaississantes. En outre, ils permettent avantageusement de mettre en suspension des principes actifs cosmétiques ou des principes actifs thérapeutiques. Enfin, les carbomères ne sont pas toxiques et sont jugées sûres d'emploi. Les carbomères sont également connus sous le nom INCI « Carbomer ».

Avantageusement, les carbomères permettent d'atteindre une viscosité de la composition comprise entre 20 000 et 30000 cps. Ainsi, les carbomères permettent d'obtenir une gélification satisfaisante au sens de la présente invention et une composition gélifiée avec un aspect visuel transparent.

Selon un mode de réalisation, particulièrement adapté, l'agent gélifiant représente entre 0,5 et 3% en poids du poids total de la composition, permettant ainsi d'obtenir une viscosité comprise entre 20 000 et 30000 cps.

### Procédé

L'extrait constituant ou contenu dans le principe actif selon l'invention peut être obtenu par tout procédé comprenant au moins une étape d'extraction, préférentiellement une macération hydroalcoolique, plus préférentiellement la solution hydroalcoolique est du vin blanc présentant un degré alcoolique compris entre 11 et 13°, préférentiellement 12°.

Selon un mode de réalisation particulièrement adapté, le principe actif selon l'invention est obtenu par la mise en oeuvre des étapes suivantes :
a. Mélanger les fruits de *Juniperus Communis, Rosmarinus Officinalis,* et le vin blanc
b. Macération dans le vin blanc à une température comprise entre 25° et 60°C,
c. Filtration du macérat obtenu, formant l'extrait
d. Eventuellement concentration et/ou purification de l'extrait obtenu à l'étape précédente.

Préférentiellement, la macération est mise en oeuvre à une température comprise entre 25 et 60°C, plus préférentiellement 50°C pendant au moins 20 minutes.

Eventuellement, le procédé comprend une étape de filtration à partir du macérat obtenu pour éliminer les particules solides et les éventuels dépôts. Ainsi, cette étape de filtration permet la purification du macérat obtenu.

Le produit obtenu à ce stade peut éventuellement être encore concentré et/ou purifié, préférentiellement par des étapes d'ultrafiltrations successives à travers des filtres de porosité différentes, en conservant les filtrats à chaque étape et/ou par une méthode de type chromatographique.

Le produit obtenu après filtration, avant ou après concentration et filtration stérilisante, est un extrait de *Juniperus Communis* et *Rosmarinus officinalis* et de vin blanc, et constitue une première forme du principe actif selon l'invention, se présentant alors sous forme liquide.

Le procédé peut, en outre, comprendre l'ajout d'un agent gélifiant pour obtenir une composition comprenant le principe actif selon l'invention sous une forme gélifiée. Cette forme étant particulièrement adaptée pour une application cutanée.

### Utilisation

Le principe actif selon l'invention ou la composition selon l'invention vise à soulager les douleurs liées à la sciatique, mais également diminuer l'inflammation cutanée. Dans ce contexte, la présente invention se rapporte au principe actif selon l'invention ou la composition comprenant ledit principe actif pour son utilisation comme médicament.

Préférentiellement, pour son utilisation pour lutter contre l'inflammation, notamment l'inflammation cutanée, et/ou pour lutter contre les douleurs, en particulier les douleurs de la sciatique.

Selon un autre objet, le principe actif selon l'invention ou la composition vise également à améliorer la cicatrisation cutanée.

Lorsque le principe actif selon l'invention ou la composition comprenant ledit principe actif est destiné à des personnes ne souffrant pas de douleurs sciatiques ou ne présentant pas de phénomènes inflammatoires, la présente invention vise également des utilisations cosmétiques. Ainsi, selon un autre objet, la présente invention porte également sur une utilisation cosmétique du principe actif selon l'invention ou de la composition comprenant ledit principe actif, pour ses effets apaisants, relipidants, mais également pour améliorer la fonction barrière, en particulier la barrière cutanée.

L'invention est à présent illustrée par des exemples non limitatifs d'extrait et de compositions et par des résultats d'efficacité.

### Exemples

### Exemple 1 - Extrait selon l'invention

Un premier extrait est réalisé à partir de :
a. 94.5%, en poids du poids total, de vin blanc biologique à 12% d'alcool.
b. 4,5%, en poids du poids total, de romarin frais
c. 1%, en poids du poids total, de baies de genièvre frais

Une macération des baies et du romarin est faite dans le vin blanc pendant 30 minutes à 50°C à l'aide d'une plaque chauffante. La température est surveillée régulièrement avec un thermomètre pour contrôler si aucune variation ne survient.

Une légère agitation est mise en oeuvre sous défloculeuse pour permettre un mélange homogène des différents ingrédients. Une fois le temps écoulé, une première filtration est réalisée à l'aide d'un entonnoir et d'un filtre tel qu'un papier sopalin. Un glaçage est ensuite réalisé au réfrigérateur pendant 48 heures, suivi d'une seconde filtration avec le même matériel afin de limiter les dépôts. L'extrait obtenu est alors sous forme liquide et constitue une première forme du principe actif selon l'invention.

### Exemple 2 - Extrait hors invention

Un second extrait est préparé à partir de :
a. 94.5%, en poids du poids total, d'éthanol à 97% dilué avec de l'eau déminéralisée pour avoir un degré à 12%.
b. 4,5%, en poids du poids total, de romarin frais
c. 1%, en poids du poids total, de baies de genièvre frais

Le protocole de préparation est identique à celui de l'exemple 1. Toutefois, l'extrait ne présente pas l'efficacité recherchée. Ainsi, la macération hydro-éthanolique ne permet pas d'obtenir un extrait selon la présente invention.

### Exemple 3 - Extrait hors invention

L'extrait selon l'exemple 3 est préparé à partir des ingrédients de l'exemple 1. L'extrait selon l'exemple 3 diffère en ce que la macération est réalisée à température ambiante pendant 48h. Toutefois, là encore l'extrait ne présente pas l'efficacité recherché. L'extraction à température ambiante ne permet pas d'extraire une quantité suffisante de principe actif, par conséquent, l'efficacité est insuffisante.

### Exemple 4 - Extrait hors invention

L'extrait selon l'exemple 4 est préparé à partir des ingrédients de l'exemple 2. L'extrait selon l'exemple 4 diffère en ce que la macération est réalisée à température ambiante pendant 48h. Comme pour l'extrait selon l'exemple 2, l'extrait ne présente pas l'efficacité recherchée.

### Exemple 5 - Composition selon l'invention

Un premier exemple de composition selon l'invention a été préparé, celui-ci comprend le principe actif selon l'invention, à savoir 95,3 % de l'extrait selon l'exemple 1 et 2% de gélifiant, à savoir un carbomère, en poids du poids total de la composition. Le protocole est le suivant. On mélange l'extrait selon l'exemple 1 avec le gélifiant. Puis on ajoute 0,7% de conservateur et 2% d'une solution de soude à 30%.

La solution de soude à 30% a été ajoutée dans les essais comprenant le carbomère afin de le neutraliser et permettre sa polymérisation. Plus le pourcentage et le pH seront élevés et plus la viscosité sera grande.

### Exemple 6 - Composition hors invention

Un second exemple de composition a été préparé, celui-ci comprend le principe actif selon l'invention, à savoir 98.8% de l'extrait selon l'exemple 1 et 0.5% de gélifiant, à savoir la gomme de xanthane. Le protocole est le suivant. On mélange l'extrait selon l'exemple 1 avec le gélifiant. Puis on ajoute 0,7% de conservateur.

### Exemple 7 - Essai de gélification

Les inventeurs ont comparé plusieurs gélifiants à différentes concentrations.

Les résultats sont présentés dans le tableau 1 ci-après.

**[Tableau 1]**

| **Code essai** | **Gélifiant** | **%** | **Résultats** |
|---|---|---|---|
| Extrait selon l'exemple 1 | Carbomère | 0.2% | Gélification faible, gel transparent |
| Extrait selon l'exemple 1 | Carbomère | 0.5% | Gélification moyenne, gel transparent |
| Extrait selon l'exemple 1 | Gomme de xanthane | 0.2% | Pas de gélification, gel trouble |
| Extrait selon l'exemple 1 | Gomme de xanthane | 0.5% | Gélification faible, gel trouble |

Les inventeurs ont ainsi constaté que la gomme de xanthane n'est pas un gélifiant satisfaisant étant donné qu'il trouble le gel obtenu et que l'étalement du produit est non satisfaisant, collant et peu agréable. A l'inverse, le carbomère à 0,5% permet d'obtenir un gel ayant des propriétés satisfaisantes pour le consommateur.

Les inventeurs ont ensuite testé le carbomère à 0,9% et 1% en poids du poids total de la composition avec l'extrait selon l'exemple 1 et l'extrait selon l'exemple 3.

Lorsque la composition comprend l'extrait selon l'exemple 1 et le carbomère à hauteur de 1%, en poids du poids total de la composition, celle-ci présente une viscosité de 43 000 cp.

Lorsque la composition comprend l'extrait selon l'exemple 3 et le carbomère à hauteur de 1%, en poids du poids total de la composition, celle-ci présente une viscosité de 46 800 cp.

Enfin, lorsque la composition comprend l'extrait selon l'exemple 1 et le carbomère à hauteur de 0,9%, en poids du poids total de la composition, celle-ci présente une viscosité de 22 450 cp.

Ainsi, le gélifiant doit être présent à hauteur d'au moins 0,5%.

### Exemple 8 - Etude de l'efficacité de l'extrait selon l'exemple 1 sur les douleurs de la sciatique.

L'étude a été réalisée auprès de 16 personnes souffrant de douleurs liées à la sciatique. Les participants inclus dans cette étude sont des personnes des deux sexes présentant des douleurs liées à la sciatique confirmées cliniquement ou non, tels que décrit dans le tableau 2 ci-après.

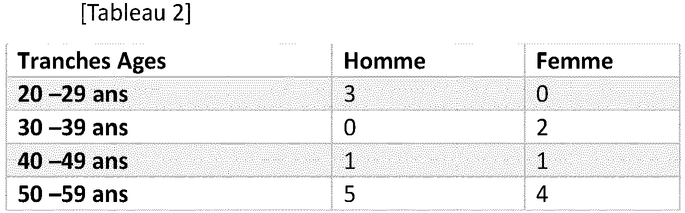

Le principe actif selon l'exemple 1 formulé sous forme de gel a été testé par l'ensemble des participants pendant une crise de sciatique selon le protocole suivant, à savoir trois applications topiques par jour pendant une semaine.

Parmi les participants, 88% ont affirmé souffrir de sciatiques, également confirmé cliniquement par un praticien lors d'un diagnostic de sciatique. En outre, 63% des participants souffrent de crises de sciatiques au moins 1 fois par mois, dont 12% en souffrent 1 fois par semaine. La douleur ressentie pendant les crises, se situe au moins à 7 sur une échelle de douleur progressive de 1 à 10. Enfin, cinq personnes affirment ressentir des douleurs insupportables.

Après application du principe actif selon l'exemple 1 par l'ensemble des participants, il en ressort que 94% n'ont constaté aucune irritation liée à l'application du produit. En outre, aucun participant n'a constaté de rougeur, d'apparition de boutons ou de sensations de tiraillement de la peau lié à l'application du produit.

Le principe actif selon l'invention est donc très bien accepté par l'ensemble des participants.

Enfin, les résultats d'efficacité du produit sont les suivants, à savoir 75% des participants ont ressenti une diminution immédiate de la douleur après une seule application dudit principe actif selon l'invention. Par application, il faut comprendre le suivi du protocole entre une journée et une semaine. Ainsi, ces mêmes personnes considèrent qu'il n'était pas nécessaire d'attendre plusieurs applications pour en ressentir les bénéfices.

100% des participants ont constaté une diminution des douleurs pendant le traitement d'une semaine, en particulier :
- 2 personnes ont constaté cette diminution après une seule journée, soit une diminution immédiate des douleurs,
- 7 personnes ont constaté cette diminution après 4 jours,
- 7 personnes ont constaté cette diminution après 1 semaine.

Enfin, 94% des participants ont confirmé que l'utilisation du principe actif selon l'invention a stoppé la crise de sciatique, ne ressentant plus de douleurs après application du produit. Pour l'ensemble des participants, la crise a été stoppée selon la même durée que l'arrêt des douleurs : soit
- 2 personnes ont constaté cet arrêt de crise immédiatement après une journée
- 7 personnes ont constaté cet arrêt de crise après 4 jours.
- 7 personnes ont constaté cet arrêt de crise après 1 semaine

Ainsi, le principe actif selon la présente invention est très bien toléré par les participants. En effet, aucun effet secondaire sur la peau, ni irritations (sauf 1 personne), ni rougeurs, ni apparition de boutons ou de sensation de tiraillement n'est apparu.

L'acceptance et l'utilisation du principe actif selon l'invention sont donc très positives. Il en ressort que la texture du gel est assez épaisse, permettant ainsi de s'étaler facilement, procurant un massage agréable, tout en laissant la peau douce et confortable. En outre, plus de la moitié des participants ont apprécié son odeur.

Concernant l'efficacité, 100% des participants ont ressenti une diminution des douleurs pendant une crise de sciatique, avec des effets constatés, immédiatement après 1 journée (12%), après 4 jours (44%) et après 1 semaine (44%).

L'ensemble des participants a pu constater que la crise de sciatique s'est arrêtée dans les mêmes durées, signifiant que les douleurs n'étaient plus présentes les jours suivants.

Au regard de ces résultats, 100% des participants confirment l'efficacité du principe actif selon l'invention pour lutter contre les douleurs de la sciatique.

### Exemple 9 - Etude de l'effet de l'extrait selon l'exemple 1 par analyse protéomique - Evaluation de la toxicité

Les essais et l'analyse protéomique ont été réalisées sur explants de peau. Les explants de peau sont couramment utilisés comme modèle dans l'étude des effets des produits cosmétiques sur la peau. En effet, en culture, ils imitent très bien les propriétés globales (biochimiques et physiologiques) de la surface de la peau. L'expérience a été effectuée sur 3 échantillons par condition avec une application du produit sur 5 jours matin et soir. L'extraction et le dosage protéique ont été réalisés en appliquant les procédures appropriées. Sur un total de 4162 protéines identifiées par spectrométrie de masse, l'annotation, la classification par catégorie fonctionnelle et l'enrichissement en protéines ont été analysés sur 2430 protéines filtrées.

Toute d'abord, l'innocuité de l'extrait est vérifiée par un essai sur un modèle cellulaire connu, à savoir les kératinocytes. La vitalité cellulaire (dosage de l'Adénosine Tri Phosphate : ATP intracellulaire) et la viabilité cellulaire (dosage de l'activité Lactate Déshydrogénase : LDH sécrétée) des kératinocytes épidermiques humains ont été analysées.

Le protocole est le suivant. L'extrait a été appliqué à des dilutions allant de 1% à 50% dans le milieu de culture pendant 24h sur des kératinocytes cultivés en monocouche. Des kératinocytes humains normaux, adultes, ont été cultivés et maintenus en monocouche à une confluence inférieure à 75% dans le milieu de culture adapté au kératinocytes Les kératinocytes ont été cultivés à 37ºC, 5% CO2. Les cellules ont été cultivées avec une combinaison de trypsine purifiée et d'inhibiteurs de trypsine/BSA et ont été utilisées jusqu'à passage 6. L'extrait selon l'invention a été appliqué à des dilutions de 1%, 5%, 10%, 15%, 20% et 50% dans le milieu de culture pendant 24h.

La teneur en ATP a été mesurée à l'aide d'un test CellTiter-Glo^{®} (Promega) selon les instructions du fabricant.

L'activité Lactate déshydrogénase (LDH) a été déterminée à partir du milieu de culture qui a été recueilli à la fin du traitement. La cytotoxicité a été mesurée en utilisant le test LDH-Glo^{®} (Promega). Un contrôle positif a été appliqué en traitant les cellules avec 0,1% v/v (eau) de Triton-X100 (Sigma-Aldrich). Les protocoles ont été exécutés conformément aux instructions du fabricant.

L'ATP est la principale forme d'énergie dans les cellules. Elle peut donc être utilisée comme outil d'évaluation de la toxicité, car toutes les cellules ont besoin d'énergie pour rester en vie et remplir leurs fonctions. L'ATP a été mesurée après traitement dose-réponse (différentes concentrations) avec l'extrait selon l'invention pendant 24h, en comparaison avec des cellules non traitées. L'Antimycine A (AMA) empêche la production d'ATP et est donc un contrôle positif de l'essai.

Les résultats sont présentés en Fig. 1.

Les inventeurs ont ainsi observé une diminution des niveaux d'ATP à partir d'une concentration de 2% dans les cellules traitées avec les actifs pendant 24h, par rapport aux cellules témoins non traitées.

Dans un second temps, des essais basés sur la LDH ont été réalisés pour déterminer le pourcentage de cellules mortes dans des conditions données. En effet, la LDH intracellulaire est libérée dans le milieu de culture lors de la mort cellulaire, et la mesure de l'activité hors de la cellule de la LDH est donc un test classique pour également estimer la cytotoxicité. Combiné aux mesures de l'ATP, ce test est utile pour mesurer le stress et la mort cellulaire. L'activité de la LDH dans les milieux de culture a été mesurée après un traitement dose-réponse avec l'extrait pendant 24h. Les résultats sont présentés en Fig. 2.

Comme le montre la Fig. 2, le contrôle positif (Triton X-100) induit une forte augmentation de la libération de LDH et l'extrait induit la libération de LDH à partir de 2-5%.

Ainsi, ces résultats confirment que l'extrait à une concentration inférieur ou égal à 1% pendant 24h n'induit pas de cytotoxicité ou de mort cellulaire Les actifs peuvent donc être considérés sans danger à une concentration inférieure ou égale à 1%.

### Exemple 10 - Etude de l'effet de l'extrait selon l'exemple 1 par analyse protéomique - Evaluation de l'effet réparateur et cicatrisant

L'inflammation et la cicatrisation sont des processus complexes et hautement coordonnés. Ces processus impliquent une série d'évènements cellulaires et moléculaires qui travaillent ensemble pour réparer les tissus endommagés, contrôler l'inflammation et restaurer la fonction de barrière de la peau.

L'inflammation dans l'épiderme, la couche la plus superficielle de la peau, est une réponse immunitaire localisée qui peut se produire en réponse à une blessure, une infection ou d'autres formes de stress tissulaire. Il s'agit d'un processus complexe impliquant divers mécanismes moléculaires, tels que la reconnaissance d'une lésion, la libération de cytokines (messagers chimiques de l'inflammation) et l'activation de voies de signalisation inflammatoires.

La réparation de la peau, en particulier de l'épiderme, est un processus biologique visant à restaurer l'intégrité de la barrière cutanée après un dommage. Bien que l'inflammation soit un mécanisme nécessaire de défense, elle doit être étroitement régulée afin d'éviter l'inflammation chronique, qui peut entraîner des lésions tissulaires et des cicatrices prononcées. Ce processus de réparation implique une série de mécanismes moléculaires et cellulaires, tels que la prolifération des kératinocytes, l'épithélialisation et la reconstitution des composants essentiels tels que les lipides et le collagène. Or, certaines protéines peuvent promouvoir, mais également inhiber, la cicatrisation.

Les résultats sont présentés en Fig. 3. L'effet anti-inflammatoire est décrit dans le panel A et l'effet cicatrisant dans le panel B.

Les inventeurs ont ainsi observé que l'extrait selon l'exemple 1 est capable d'induire une augmentation de 20% de l'abondance des protéines impliquées dans l'anti-inflammation (panel A). En outre, les inventeurs ont constaté une augmentation des protéines impliquées dans la migration des cellules et la régulation de l'inflammation telle que :
- ALCAM (Activated Leukocyte Cell Adhesion Molécule), qui facilite le mouvement des cellules vers le site à régénérer.
- SLPI (inhibiteur de la protéase des leucocytes sécrétoires), ayant des propriétés anti-inflammatoires qui joue un rôle dans la cicatrisation également, en favorisant une réponse immunitaire contrôlée qui soutient la réparation des tissus tout en minimisant l'inflammation excessive.

Enfin, les inventeurs ont également constaté que l'extrait selon l'invention est capable de réduire de 60% l'abondance des protéines susceptibles d'empêcher la réparation correcte des plaies (panel B), telles que :
- la stromelysine-1, également connue sous le nom de métalloprotéinase matricielle 3 (MMP3), qui dégrade les composants de la matrice extracellulaire
- la Psoriasin (S100A7) qui est connue pour être sur-exprimée dans les peaux atopiques et en cas de psoriasis,
- le facteur nucléaire NFκB p105 (NFκB1), dont une suractivation peut conduire à une inflammation prolongée. En excès, il peut entraver la formation de nouveaux tissus.

Les résultats démontrent ainsi une action réparatrice liée à une amélioration du renouvellement cellulaire et à un contrôle de l'inflammation.

### Exemple 11 - Etude de l'effet de l'extrait selon l'exemple 1 par analyse protéomique - Evaluation de l'effet apaisant

Les espèces réactives de l'oxygène (ROS) sont des radicaux libres de l'oxygène, qui peuvent générer un stress oxydatif. Les ROS peuvent être générés naturellement ou suite à une exposition à des facteurs externes tels que la lumière UV du soleil, la pollution de l'air ou d'autres agressions environnementales. Un niveau excessif de ROS peut causer un stress oxydatif, qui peut endommager les cellules, et contribuer au vieillissement cutané prématuré ou à des problèmes de peau. Les ROS font partie intégrante de la fonction cellulaire normale et sont équilibrés par des mécanismes intracellulaires qui réduisent leurs effets nocifs. Des actifs peuvent atténuer les effets néfastes des ROS en améliorant l'équilibre des ROS dans les cellules de la peau. Le déséquilibre oxydatif de la peau joue donc un rôle majeur dans le vieillissement prématuré.

Les résultats sont présentés en Fig. 4. Ceux-ci montrent que l'extrait selon l'exemple 1 augmente de près de 10% l'abondance de protéines impliquées dans la gestion de l'équilibre oxydatif (balance redox), telles que les protéines MGST1 (Microsomal Glutathione S-Transferase 1) et la péroxydase, qui protègent les cellules de la peau des dommages causés par les ROS. L'extrait agit ainsi comme un "bouclier" en neutralisant ces molécules nocives, favorisant ainsi la santé de la peau (Panel A).

En outre, l'autophagie, est un processus cellulaire qui permet aux cellules de recycler les composants défectueux de la cellule pour survivre à des périodes de stress. Une peau saine présente un taux de renouvellement cellulaire élevé car elle se défend continuellement contre les agressions de l'environnement. Dans ce cas, la peau s'appuie sur l'autophagie pour éliminer les composants cellulaires endommagés et ainsi se renouveler sans activer le système immunitaire. Ainsi, l'autophagie fait le ménage pour assurer la survie, le fonctionnement cellulaire, l'homéostasie et la tolérance immunitaire.

Les résultats (Panel B) montrent que l'extrait augmente de près de 20% l'abondance de protéines impliquées dans l'autophagie, telle que la protéine SQSTM1 (Séquestosome-1), également connu sous le nom de p62, qui facilite l'élimination des composants cellulaires endommagés ou nocifs afin d'assurer le bon fonctionnement des cellules de l'épiderme.

Or, les inventeurs ont observé que l'extrait selon l'invention permet d'améliorer la balance redox (équilibre oxydatif) et l'autophagie, et par conséquent de présenter une action apaisante sur la peau.

### Exemple 12 - Etude de l'effet de l'extrait selon l'exemple 1 par analyse protéomique - Evaluation de l'effet relipidant et énergisant

Les lipides dans la peau jouent un rôle essentiel pour maintenir la bonne santé et l'apparence de notre peau, ceux-ci ayant de nombreuses fonctions, à savoir : un rôle de barrière protectrice, d'hydratation, de souplesse, d'isolation thermique, ainsi que de stockage d'énergie. Les lipides regroupent différentes molécules, comme les acides gras, les phospholipides.

Le métabolisme des lipides (Panel B) et des mitochondries (Panel A) sont étroitement liés. En effet, les lipides peuvent influencer la fonction mitochondriale. Par exemple, les lipides servent de substrat pour produire de l'énergie dans la mitochondrie. Par conséquent, il est d'intérêt d'observer le rôle des protéines suivantes :
- LPCAT5 (Lysophospholipid Acyltransferase 5) protéine impliquée dans la synthèse de phospholipides,
- ACSL3 (Fatty Acid CoA Ligase) qui est spécifiquement impliquée dans l'activation des acides gras à longue chaîne en les convertissant en leurs dérivés acyl-CoA correspondants. Cette étape d'activation est nécessaire pour l'incorporation ultérieure des acides gras dans diverses voies métaboliques, notamment la production d'énergie.

Les résultats sont présentés en Fig. 5.

Les inventeurs ont alors constaté que l'extrait selon l'exemple 1 augmente le métabolisme lipidique (Panel B) afin de produire de l'énergie. En particulier, les résultats montrent que l'extrait augmente de près de 7% l'abondance de protéines impliquées dans le métabolisme mitochondrial (Panel A), notamment la production d'énergie, telles que la protéine NDUFS7 (NADH Dehydrogenase [Ubiquinone] Iron-Sulfur Protein 7), la protéine NNT (NAD(P) Transhydrogenase, Mitochondrial) et la protéine ATP51 (ATP Synthase Subunit f, Mitochondrial). Ces protéines sont notamment impliquées dans la phosphorylation oxydative (Oxphos), l'étape finale de la respiration cellulaire, où la majorité de l'ATP est générée.

Ainsi, ces résultats démontrent une action énergisante et relipidante étroitement liée à une adaptation des métabolismes lipidique et mitochondrial.

L'extrait selon l'invention montre ainsi :
- une activité réparatrice de la peau à travers une diminution des protéines inhibitrices de la cicatrisation, démontrant notamment une meilleure régulation et vitesse de la réparation cutanée ainsi qu'une amélioration du renouvellement cellulaire et du contrôle de l'inflammation.
- une activité apaisante à travers l'analyse des voies de régulation de la balance Redox et de l'autophagie qui régulent l'équilibre des espèces réactives de l'oxygène (ROS) et le stress. Elles sont impliquées dans le maintien et le renouvellement de la barrière cutanée, ce qui contribue à maintenir le confort de la peau. Elles régulent également l'expression de certaines molécules anti-inflammatoires et peuvent aider à prévenir une inflammation excessive de la peau, diminuant ainsi les sensations d'inconfort.
- une activité énergisante et relipidante. En effet, lorsque les cellules vieillissent, les mitochondries qu'elles contiennent vieillissent également, et leur capacité à produire de l'énergie diminue. Ce déclin de la production d'énergie peut entraîner une augmentation de la production des espèces réactives de l'oxygène (ROS), qui peuvent affecter la barrière cutanée. La capacité de la mitochondrie à maintenir les niveaux d'énergie permet également de maintenir un métabolisme lipidique équilibré, sain et actif qui améliore la barrière cutanée.

Ainsi, les résultats démontrent que l'extrait selon l'invention présente des effets apaisants, anti-inflammatoires, énergisants et relipidants.

## Revendications

1. Principe actif comprenant un extrait hydroalcoolique, ledit extrait comprenant un mélange constitué essentiellement de :
a. Fruits de *Juniperus Communis,*
b. *Rosmarinus Officinalis,* et
c. vin blanc à 12°C

2. Principe actif selon la revendication précédente, **caractérisé en ce que** les fruits de *Juniperus Communis* représentent entre 0,5 et 2% en poids du poids total du mélange.

3. Principe actif selon l'une des revendications précédentes, **caractérisé en ce que** *Rosmarinus Officinalis* représente entre 1,5 et 8% en poids du poids total du mélange.

4. Principe actif selon l'une des revendications précédentes, **caractérisé en ce que** le vin blanc représente entre 90 et 98% en poids du poids total du mélange.

5. Principe actif selon l'une des revendications précédentes, **caractérisé en ce que** l'extrait est obtenu par un procédé d'extraction comprenant les étapes suivantes :
a. Mélanger des fruits de *Juniperus Communis, Rosmarinus Officinalis,* et du vin blanc,
b. Macération dans le vin blanc à une température comprise entre 25° et 60°C,
c. Filtration du macérat obtenu, formant l'extrait,
d. Eventuellement concentration et/ou purification de l'extrait obtenu à l'étape précédente.

6. Composition comprenant au moins un principe actif selon l'une des revendications précédentes et au moins un excipient acceptable.

7. Composition selon la revendication précédente, **caractérisé en ce que** l'excipient est un gélifiant, celui-ci est compris entre 1 et 3%, en poids du poids total de la composition.

8. Composition selon l'une des revendications 6 ou 7, ladite composition se présente sous une forme adaptée à une application topique.

9. Composition selon la revendication précédente, ladite composition se présente sous une forme choisie parmi un gel, une crème, un sérum, un masque et une lotion.

10. Principe actif selon l'une des revendications 1 à 5 ou composition selon l'une des revendications 6 à 9, pour son utilisation comme médicament.

11. Principe actif selon l'une des revendications 1 à 5 ou composition selon l'une des revendications 6 à 9, pour son utilisation pour lutter contre les douleurs de la sciatique.

12. Principe actif selon l'une des revendications 1 à 5 ou composition selon l'une des revendications 6 à 9, pour son utilisation pour lutter contre l'inflammation.

13. Principe actif selon l'une des revendications 1 à 5 ou composition selon l'une des revendications 6 à 9, pour son utilisation pour améliorer la cicatrisation cutanée.

14. Utilisation cosmétique d'un principe actif selon l'une des revendications 1 à 5 ou d'une composition selon l'une des revendications 6 à 9, pour ses effets apaisants, relipidants

15. Utilisation cosmétique d'un principe actif selon l'une des revendications 1 à 5 ou d'une composition selon l'une des revendications 6 à 9, pour améliorer la barrière cutanée.
